# EUROPEAN PATENT SPECIFICATION

(11) **EP 3 164 143 B1**
(45) Date of publication and mention of the grant of the patent: **26.02.2020**
(21) Application number: 15807579.6
(22) Date of filing: 10.06.2015
(51) Int. Cl.: A61K 35/74, A61P 1/00, C12Q 1/04, C12Q 1/68, A61K 35/747, A61K 31/702, A61K 45/06, C12Q 1/06, A23L 33/135, A23L 33/21, A61K 35/745

(54) **MICROBIOMARKER FOR CELIAC DISEASE AND A RELATED PRODUCT**
MIKROBIOMARKER FÜR ZÖLIAKIE UND VERWANDTE PRODUKTE
MICRO-BIOMARQUEUR POUR MALADIE COELIAQUE ET PRODUIT ASSOCIÉ

(30) Priority: 11.06.2014 FI 20145537
(43) Date of publication of application: 10.05.2017
(73) Proprietor: Gut Guide Oy, FI-24800 Halikko (FI)
(72) Inventor: MÄYRÄ, Annika, FI-24800 Halikko (FI); MUNUKKA, Eveliina, FI-24800 Halikko (FI)
(74) Representative: Kolster Oy Ab
(86) International application number: PCT/FI2015/050411
(87) International publication number: WO 2015/189472

(56) References cited:
- EP-A1- 2 236 598
- WO-A1-2011/043654
- WO-A1-2013/054002
- WO-A1-2013/191845
- WO-A1-2014/070014
- US-B2- 7 862 808
- SCHOSTER A ET AL: "In vitro inhibition of Clostridium difficile and Clostridium perfringens by commercial probiotic strains", ANAEROBE, vol. 20, April 2013 (2013-04), pages 36-41, XP002776546,
- TURSI ANTONIO ET AL: "High prevalence of celiac disease among patients affected by Crohn's disease.", INFLAMMATORY BOWEL DISEASES JUL 2005, vol. 11, no. 7, July 2005 (2005-07), pages 662-666, XP055433705, ISSN: 1078-0998
- NADAL I. ET AL.: 'Imbalance in the composition of the duodenal microbiota of children with coeliac disease.' JOURNAL OF MEDICAL MICROBIOLOGY vol. 56, no. 12, 2007, pages 1669 - 1674, XP055244420
- 'Probiotic bacteria in non-dairy food applications.' SACCO PROBIOTICS., [Online] 23 September 2015, XP008185912 Retrieved from the Internet: <URL:HTTP://WWW.SACCOSRL.IT/EN/PRODOTTI/COL TURE-PROBIOTICHE/>

## Description

### Field of the invention

The present invention relates to celiac disease. More precisely the invention relates to a microbiological product for use in improving the state of gut health of individuals suffering of celiac disease. Further the invention relates to a method for identifying a person having celiac disease by a specific gut health biomarker, Celiac Gut Index (CGI).

### Background of the invention

Celiac disease (CD) is an autoimmune disorder of the small intestine that occurs in genetically predisposed people of all ages from infancy on up. Celiac disease is caused by a reaction to gliadins, a family of related pro-line and glutamine rich protein in gluten protein, which are found in wheat (and similar proteins of the tribe Triticeae, such as barley and rye). Upon exposure to gliadin, the enzyme tissue transglutaminase modifies the protein, and the immune system of subjects prone to celiac disease reacts and cross-reacts with the small-bowel tissue, causing an inflammatory reaction. The inflammation subsequently leads to villous atrophy and interferes with the absorption of nutrients, including minerals and fat soluble vitamins. The other causative factors in celiac disease besides gluten involve host genetic background (HLA-DQ2 or DQ8 and other non-HLA genes) and environmental cofactors, such as intestinal pathogens, altered gut microbiota composition, infant-feeding practices and some immune-modulatory drugs.

The composition of gut microbiota has been demonstrated to have numerous effects on the wellbeing and health of the host (Hooper L.V., Gordon J. Science 2001; and Bäckhed, F et al. 2005, Science)*.* Dysequilibrium of the gut microbiome has been associated with several diseases, including autism, bowel disease and cancer, rheumatoid arthritis, diabetes, and obesity. Recent studies have pointed to the possible role of the gut microbiota in the development of celiac disease (Calabrò A. et al. Autoimmune Dis. 2014). It has been demonstrated that the homeostatic mechanisms that allow coexistence of the host organism and the commensal microbiota are disrupted in celiac disease. Imbalance in the composition of the duodenal microbiota of children with celiac disease has been reported (Nadal et al. Journal of Medical Microbiology, 2007). It was also demonstrated that the duodenal-mucosal microbiota of CD patients presents alterations in the diversity and abundance of different cultivable bacterial taxa (Jing Cheng et al. BMC Gastroenterol. 2013).

Typical symptoms of celiac disease include abdominal distension, vomiting, diarrhoea, weight loss, anaemia and fatigue. Recognizing celiac disease can be difficult because some of its symptoms are similar to those of other diseases related to gut imbalance. Symptoms also vary depending on a person's age and the degree of damage to the small intestine. Many adults have the disease for a decade or more before they are diagnosed. The longer a person goes undiagnosed and untreated, the greater the chance of developing long-term complications.

Diagnosis of celiac disease is done mainly using serologic tests. In patients with positive serology, a biopsy of the small intestine showing typical CD characteristics (increased number of intra-epithelial lymphocytes, elongation of the crypts and villous atrophy) is still required to confirm the diagnosis. In fact, for the past few decades, biopsy has been the only relatively reliable and diagnostically accepted path to diagnosis. The problem is that biopsies are expensive and highly invasive, whereas antibody tests would be a cheap and painless alternative, but they haven't proven themselves to be accurate enough for conclusive diagnosis.

Despite the increased knowledge on celiac disease, the only known effective treatment of celiac disease so far is a lifelong gluten-free diet. Even if the gluten-free diet appears simple in principle, it is very restrictive, costly, socially incapacitating and very often difficult to implement without medical support. As the compliance with the dietary recommendation is difficult, patients continue suffering of gastrointestinal symptoms, nutritional deficiencies, and higher health risks.

In the last years, alternative therapeutic strategies have been tested (Piscaglia, World J Stem Cells 2014). These include intraluminal therapies such as genetic modification of wheat and/or pretreatment of flours to reduce immunotoxicity, oral enzyme therapy, intraluminal binding of gluten peptides and neutralizing gluten antibodies, transepithelial treatments such as inhibition of intestinal permeability through zonulin receptor antagonists and subepithelial actions such as TG inhibitors, gluten peptides that downregulate innate responses, HLA-DQ2 inhibitors, CCR9 and integrin antagonists, IL-15 antagonists, anti-IFN-y antibody, anti-CD3, anti-CD4 and anti-CD25 antibodies. Such approaches have been tested in experimental models and in small clinical trials with inconclusive results overall in terms of efficacy and safety profile.

US2013121976 discloses bacterial strains that are suitable for use in prevention and/or treatment of celiac disease. These strains of *Lactobacillus* and *Streptococcus* which have a capacity to degrade gliadin peptides involved in celiac disease, peptide degrading activity being stable under low pH and in the presence of mammalian digestive enzymes. EP2236598 discloses microorganisms, especially *Bifidobacterium,* which are capable of being used for treatment or prevention of immune-mediated diseases, such as celiac disease.

Currently there are no easily implemented methods available for an early diagnostics of celiac disease. Biomarkers for CD are urgently needed since future disease modifying therapies should be initiated as early as possible in the disease process to maximize their effect. Moreover, despite the several alternative therapeutic strategies, there still remains a need for an effective and non-costly treatment option.

### Brief description of the invention

An object of the present invention is thus to provide an efficient method for aiding diagnosis of celiac disease and an option for an effective treatment of celiac disease.

The objects of the invention are achieved by a product which is to be used for modulation of gut microbiota composition of diagnosed celiac disease patients or people suspected to have celiac disease. The product may also serve as a dietary supplement for individuals having gluten-free diet.

The present invention relates to a product for use in improving gut health of individuals suffering from or susceptible to suffer from celiac disease, the product being as defined in claim 1. The product increases the ratio of *Bifidobacteria* and *Faecalibacterium* to GNPB in the gut.

The objects of the invention are further achieved by a method for detecting celiac disease in an individual, the method comprising determining *in vitro* the ratio of the total amount of *Bifidobacteria* and *Faecalibacterium* to gram-negative Proteobacteria (GNPB) in a fecal sample of a individual, whereby a decreased ratio of the total amount of *Bifidobacteria* and *Faecalibacterium* to GNPB compared to the reference value from healthy individuals is indicative of celiac disease in said individual.

The preferred embodiments of the invention are disclosed in the dependent claims.

The present invention is based on the study made on human material, in which it was discovered that gut microbiota composition of individuals elicited a celiac disease diagnose have significant differences in some bacterial groups/genera compared to reference data obtained from healthy individuals. Specifically changes in the ratio of the total amount of *Bifidobacteria* and *Faecalibacterium,* which are included in the commensal gut microbiota in the intestines, to the amount of GNPB in the intestines correlates negatively. The above correlations enable diagnostic methods for aiding diagnosis of celiac disease by determining the relative proportion of *Bifidobacteria* and *Faecalibacterium* to GNPB. Furthermore based on the easy determination of the gut microbiota composition a preferable dietary product for modifying the gut microbiota composition was found. The product was shown to have clear, beneficial effect on the gut microbiota composition of the celiac disease patients. It decreases the symptoms and irritation at the gut microvillus. The new product of the invention provides thus a natural and safe manner for the treatment of celiac disease.

The present invention provides also a novel approach for the diagnostics of celiac disease. The method aiding diagnosis of celiac disease is rapid, non-invasive and easy to use. The ratio of *Bifidobacteria* and *Faecalibacterium* to GNPB in the gut can be used as a microbiomarker of the gut health. The method of the invention may be used at any point during the nutritional counseling of celiac patient. The method allows a continuous follow-up of patients without any expensive, time consuming and painful operation.

The present invention provides a considerable advantage of enabling the individuals having a risk of CD being diagnosed at an early stage. Once diagnosed at an early stage as belonging to the risk group, the onset of CD in the individual can be prevented by modifying the community structure of the gut microbiota.

The combination of the method and the product of the invention enable development of personalized treatment and possibly personalized dietary guidance. An advantage is that the present method and product can be easily implemented.

### Detailed description of the invention

As used herein the term "'celiac disease'" encompasses a spectrum of conditions caused by varying degrees of gluten sensitivity, including a severe form characterised by a flat small intestinal mucosa (hyperplastic villous atrophy) and other forms characterised by milder symptoms.

The individual used herein in the context of diagnosis or therapy is human. The individuals may have symptomatic or asymptomatic celiac disease or be suspected of having it. They may be on a gluten free diet. They may be susceptible to celiac disease, such as a genetic susceptibility.

Gut microbiota is an extremely complex ecosystem (over 1000 species, in total more than 10¹⁴ bacteria). A change in the percentual proportion of one bacteria group also changes the percentual proportions of other bacteria groups significant to health. For this reason, it is important to deal not only with the change in individual bacteria groups but also with the change in the whole system.

In the method of the present invention the probability of a subject having celiac disease (CD) is determined by a method wherein a sample is obtained from a subject; the relative abundances of one or more microbial taxa in the sample are measured; and the probability of the subject having CD is determined based on the measured relative abundances of one or multiple microbial taxa in the sample. Specifically, in the present invention, the composition of the bacterial system of gut is represented by a simple and understandable Celiac Gut Index (CGI) and the index is utilized in a method for aiding diagnosis of celiac disease. The CGI index is calculated from the percentual proportions of three bacteria groups and genera significant to gut health, i.e. by dividing the sum of the amount of *Bifidobacteria* and *Faecalibacterium* in a sample by the amount of gram-negative *Proteobacteria* (GNPB). The change in the ratio of the total amount of *Bifidobacteria* and *Faecalibacterium* to the amount of GNPB in the intestines correlates negatively with celiac disease. The expression "correlates negatively" means that when one variable increases, another one decreases. Thus, the higher the relative value of the CGI in a subject is, the lower is the likelihood of having celiac disease.. In other words, low CGI is a specific microbiomarker for dysbiotic gut microbiota composition and an indication of celiac disease. CGI is low when the level of potentially inflammatory bacteria, GNPB, is high compared to amounts of gut protective groups of bacteria, *Bifidobacteria* and *Faecalibacterium.* In an embodiment of this invention, the CGI index value above 15 represents a healthy phenotype and the value below 10, in turn, represents a phenotype with celiac disease.

The CGI index is determined indirectly by analysing the microbiota in the intestinal contents. Normally a fecal sample of the subject is examined to quantitatively determine the *Bifidobacteria, Faecalibacterium* and GNPB and/or total bacteria by methods known per se. Preferably, the proportion of these bacteria in the total bacteria is determined by a method based on 16S rRNA hybridisation, DNA staining and flow cytometry (FCM-FISH), which allows different gut bacteria groups to be determined rapidly and reliably. The relative abundance of different gut bacteria groups can also be measured using techniques based on DNA sequencing, quantitative PCR, DNA microarray, or any other suitable method. In one embodiment of the invention the gut microbiota composition, i.e. the CGI index, is determined from a non-invasive fecal sample by flow cytometry-FISH methodology.

All of the above mentioned bacteria groups belong, in the light of present knowledge to the commensal human gut microbiota. *Bifidobacteria* and *Faecalibacterium* are both known to have positive effect on the condition of gut villus. "*Bifidobacteria*" are gram-positive, immobile anaerobic bacteria that appear in the digestive tract (and belong to the *Bifidobacteriaceae* family and particularly to the *Bifidobacterium* genus). "*Faecalibacterium*" refers herein to *Faecalibacterium prausnitzii,* which is the most abundant bacterium in the human intestinal microbiota of healthy adults, representing more than 10% of the total bacterial population. *F. prausnitzii* are gram-negative anaerobic bacteria, which belong to the *Clostridium leptum* group (*Clostridium* cluster IV), belonging to phylum *Firmicutes. Proteobacteria* (gram-negative proteobacteria, GNPB) are a major group (phylum) of bacteria. They include a wide variety of pathogens, such as *Escherichia, Salmonella, Vibrio, Helicobacter,* and many other notable genera. In excessive amounts proteobacteria may potentially cause inflammation in the gut.

The CGI index can be used for monitoring a change in the composition of the gut bacteria for instance when a person has started a gluten-free diet by taking samples at different points of dietary treatment. By means of the CGI index, a customized probiotic and/or prebiotic intervention aiming at alleviating symptoms of celiac disease is possible. CGI index may serve as a preliminary microbiological biomarker i.e. microbiomarker for celiac disease in addition to genetic marker (HLA) tests from blood. The CGI index may be determined before invasive biopsies are taken from patients.

Changes in diet can induce significant changes in the gut microbiota composition. The present invention provides a novel product containing microorganisms that improves the state of health of individuals suffering or susceptible to suffer from disorders related to the ingestion of gluten, especially celiac disease and reduces the likelihood of acquiring celiac disease. The product acts on the gut microflora and changes the relative proportion of *Bifidobacteria* and *Faecalibacterium* and *Proteobacteria* (GNPB) in the intestines and, in particular, the ratio of *Bifidobacteria* and *Faecalibacterium* to GNPB. "The relative proportion of e.g. *bifidobacteria*" refers to the ratio of e.g. *Bifidobacteria* to other bacteria in the intestines, normally to the total bacteria. The product according to the invention may also have a decreasing effect on the relative proportion of *Proteobacteria* (GNPB) to the total bacteria in the intestines.

The product of the present invention contains living active bacteria in a concentrated, freeze-dried form. The product includes at least one probiotic strain or combination of strains that stimulate the growth of *Faecalibacterium* group bacteria and has an effect on villus growth and gut permeability in the intestinal tract. Preferably the product includes selected probiotic lactic acid bacteria, such as *Lactobacillus rhamnosus-,* and *Bifidobacterium* lactis-strains. *Bifidobacteria lactis* are preferably selected from strains *Bifidobacterium animalis subsp. lactis* Bb-12, *Bifidobacterium lactis* BLC1 and *Lactobacillus acidophilus.* According to one embodiment of the invention the product includes two live, bacterial strains *Lactobacillus rhamnosus SP1* (DSM 21690) and *Bifidobacterium lactis BLC1* (DSM 17741, LGM23512), both 1000 millions/dose. The product of the invention may also be any combination of above mentioned *Bifidobacteria, Lactobacilli,* prebiotic, probiotic or a product stimulating *Bifidobacteria.*

The daily dose of the bacteria is preferably 100-1000 mil bacteria. The dose may be adjusted based on the personal CGI. An increase in CGI indicates improved gut health. Higher CGI in the end of intervention indicates that the amounts so-called beneficial bacteria (*Bifidobacteria and Faecalibacterium*) and potentially inflammatory bacteria (GNPB) are more balanced.

The product of the invention may be in the form of a food composition, pharmaceutical composition, nutraceutical, or supplement. The product of the invention may be administered mixed in food or drink, for example, or separately in the form of a tablets, capsules, microcapsules, powders, solutions, pastes, etc. Food composition may be any kind of food (functional, conventional and novel), food supplement, formula for nutritional purposes, or nutraceutical and it may contain any suitable additives and excipients. The product in the form of a pharmaceutical composition may be used in treatment or prevention of celiac disease.

The increase of the amount of *Faecalibacterium* is enhanced by using in combination with probiotics some prebiotic compound."Probiotics" are live microorganisms which, when administered in adequate amounts, confer a health benefit on the host. "Prebiotics" are indigestible food ingredients that have a beneficial effect on the intestinal tract. A prebiotic through their selective metabolism is a component which is usually a carbohydrate (an oligo- or polysaccharide) and which has a selective promoting effect on the growth or activity of one or more bacterial strains in the colon. A prebiotic is preferably a fructo- or galacto-oligosaccharide, fibre, particularly cereal fibre, such as b-glucan of oat, pure oat, polydextrose, special sugar, such as isomaltulose, or for example a fatty acid, such as omega-3 fatty acid, or any mixture of these. Preferably pure oat is used. In an unpublished animal trial it was shown that feed supplemented with dehulled oat increased the amount of *Faecalibacterium* by 74 percentages in piglet feces after the 3-week intervention (GutGuide Oy, unpublished results). This result suggests that pure oat may be suitable liquid fiber source for celiac patients and could serve as an efficient prebiotic combined with probiotic strain(s).

The product stimulating *Bifidobacteria* may be a product containing propionic acid bacteria, such as *Propionibacterium freudenreichii, Propionibacterium shermanii,* and/or *Lactobacilli,* such as *Lactobacillus acidophilus, Lactobacillus rhamnosus, Lactobacillus casei* or *Lactobacillus lactis.* The bacterial strains of the product can be combined with other microorganisms and bioactive compounds to improve their protective and metabolic properties.

The present disclosure relates further to a method for treatment or prevention of CD in an individual by administering said individual an effective amount of a composition comprising a gut microbiome altering agent that, when administered to an individual, increases the CGI. "An effective amount" of a composition increasing the CGI refers to an amount sufficient for changing the relative proportion of *Bifidobacteria* and *Faecalibacterium* and GNPB in the intestines and, in particular, increasing the ratio of *Bifidobacteria* and *Faecalibacterium* to GNPB in the gut. Preferably, the composition to be administered is the product of the present invention. The term "treatment" may refer to both therapeutic treatment and prophylactic treatment or preventative measures, wherein the goal of the treatment is to slow the disease process or even stop it, or prevent CD.

### Examples

Fecal samples were collected from 34 adult Finns that have elicited a celiac diagnose (3 men, 31 women) and were already on gluten-free diet. Two samples, pre and post intervention, were collected from each participant. Pre samples were collected before the consumption of the test product and post samples after 40 day use of the test product.

The gut microbiota composition analysed from the pre samples were compared to the reference database i.e. database of gut microbiota composition of healthy Finns without any gastrointestinal disorders or diseases.

The test product included two live, bacterial strains *Lactobacillus rhamnosus SP1* (DSM 21690) and *Bifidobacterium lactis BLC1*( DSM 17741, LGM23512), both 1000 millions/dose. The bacterials strains were obtained from Sacco Ltd, Italia. In addition, a minimal amount (1 g) of fructo-oligosaccharide, (FOS) were included in the product in order to support beneficial probiotic events in the gut. All subjects consumed the product i.e. each subject served as his/her own control.

After the 40-day-period of consumption the post samples were collected and the gut microbiota composition were analysed by using a method based on whole cell, 16S rRNA *in situ* hybridisation, DNA staining and flow cytometry (Vaahtovuo J et al. J Microbiol Methods. 2005; 63:276-286). Briefly, bacteria from fecal samples were isolated from debris and fixed prior the *in situ* hybridisation. The following gut bacterial groups or genera were determined from them: *Bifidobacteria, Faecalibacterium* and GNPB including for example *Escherichia coli.* All of the above bacteria groups belong, in the light of present knowledge to the commensal human gut microbiota. The percentage proportions (of total bacteria amount) of bacteria groups were determined from the samples. Celiac Gut Index (CGI) was counted for each person by dividing the sum of percentages of *Bifidobacteria* and *Faecalibacterium* by the percentage of GNPB.

The statistical analysis has been done by IBM Statistics-program by using Student's paired t-test (Ce vs. normal and pre - post analysis).

### Results

Comparison of the gut microbiota composition of celiacs compared to reference data revealed significant differences in all the bacterial groups/genera analysed (Table 1). In addition, there is a clear significant difference in CGI between the groups (Table 1). Low CGI could serve as a new specific biomarker for dysbiotic, unbalanced gut microbiota composition that may refer to celiac disease. In celiac patients the level of potentially inflammatory bacteria (GNPB) is high compared to amounts of gut protective groups of bacteria.

**Table 1. The comparison of the gut microbiota composition between celiacs pre samples and reference data (healthy adults).**

| | Celiacs | Reference data | *P* |
|---|---|---|---|
| *Bifidobacteria* (%) | 4.7 | 7.4 | 0.003 |
| *Faecalibacterium* (%) | 5.3 | 10.0 | <0.001 |
| GNPB (%) | 2.1 | 0.9 | <0.001 |
| CGI^{≠} | 6.6 | 21.8 | <0.001 |

| | | | |
|---|---|---|---|
| CGI = Celiac Gut Index, GNPB = gram-negative proteobacteria ^{≠}CGI was calculated for each individual as described i.e. value in the Table 1 is average of those values. | | | |

The test product had clear, beneficial effect on the gut microbiota composition of the celiacs after the 40 days intervention. The amount of beneficial bacterial group *Faecalibacterium* increased significantly, and the amount of potentially, inflammatory, enteric bacteria decreased (Table 2). The overall gut microbiota composition described by the significant increase in CGI (Table 2). The significant increase of CGI indicates that improved gut health was obtained after the consumption of test product. Higher CGI in the end of intervention indicates that the amounts so-called beneficial bacteria (*Bifidobacteria* and Faecalibacterium) and potentially inflammatory are more balanced.

**Table 2. Effects of the intervention (test product for 40 days) on gut microbiota composition**

| | Pre | Post | Percentual change | *p* |
|---|---|---|---|---|
| *Bifidobacteria* (%) | 4.7 | 5.1 | 7.8 | >0.05 |
| *Faecalibacterium* (%) | 5.3 | 6.7 | 26.4 | 0.010 |
| GNPB (%) | 2.1 | 1.4 | -33.3 | <0.001 |
| CGI | 6.6 | 10.3 | 56.1 | 0.002 |

It will be obvious to a person skilled in the art that, as the technology advances, the inventive concept can be implemented in various ways. The invention and its embodiments are not limited to the examples described above but may vary within the scope of the claims.

### References

Hooper L.V., Gordon J.I., Science 2001; 292: 1115-8;
Bäckhed, F. et al. 2005. Science 307, 1915-1920
Calabrò A et al. Autoimmune Dis. 2014; 756138
Nadal I, Donant E, Ribes-Koninckx C, Calabuig M, Sanz Y. Journal of Medical Microbiology. 2007;56(12):1669-1674
Jing Cheng et al. 2013; 13: 113.
Anna Chiara Piscaglia, World J Stem Cells 2014 April 26; 6(2): 213-229
Vaahtovuo J et al. J Microbiol Methods. 2005; 63:276-286

## Claims

1. A product for use in improving gut health of individuals suffering or susceptible to suffer from celiac disease, wherein the product includes *Lactobacillus rhamnosus SP1* and *Bifidobacterium lactis BLC* -strains; and optionally prebiotics and/or a product stimulating *Bifidobacteria* and *Faecalibacterium,* or any combination of these; and wherein the product increases a ratio of the total amount of *Bifidobacteria* and *Faecalibacterium* to GNPB (gram negative Proteobacteria) when administered to an individual.

2. The product for use according to claim 1, wherein the prebiotic is selected from a group consisting of fructo- and galacto-oligosaccharides, xylo-oligosaccharides, fibres or oat.

3. A method for detecting celiac disease in an individual, the method comprising:
(a) determining a ratio of the total amount of *Bifidobacteria* and Fae*calibacterium* to gram-negative Proteobacteria (GNPB) in a fecal sample of an individual; and
(b) comparing the ratio determined in step (a) to a reference value,
wherein a decreased ratio of the total amount of *Bifidobacteria* and *Faecalibacterium* to gram-negative Proteobacteria (GNPB) compared to the reference value is indicative of celiac disease in said individual.

4. The method according to claim 3 for use in monitoring an individual's response to treatment of celiac disease.

## Patentansprüche

1. Produkt zur Verwendung bei der Verbesserung der Darmgesundheit von Individuen, die an Zöliakie leiden oder anfällig sind, daran zu leiden, wobei das Produkt Stämme von *Lactobacillus rhamnosus SP1* und *Bifidobacterium lactis BLC* umfasst; und gegebenenfalls Präbiotika und/oder ein *Bifidobacteria-* and *Faecalibacterium*-stimulierendes Produkt oder eine beliebige Kombination derselben umfasst; und wobei das Produkt ein Verhältnis der Gesamtmenge von *Bifidobacteria* und *Faecalibacterium* zu GNPB (gramnegativen Proteobakterien) bei Verabreichung an ein Individuum erhöht.

2. Produkt zur Verwendung nach Anspruch 1, wobei das Präbiotikum aus der Gruppe bestehend aus Fructo- und Galactooligosacchariden, Xylooligosacchariden, Fasern oder Hafer ausgewählt ist.

3. Verfahren zum Nachweisen von Zöliakie in einem Individuum, wobei das Verfahren umfasst:
(a) Bestimmen eines Verhältnisses der Gesamtmenge von *Bifidobacteria* und *Faecalibacterium* zu gramnegativen Proteobakterien (GNPB) in einer Fäkalprobe eines Individuums; und
(b) Vergleichen des in Schritt (a) bestimmten Verhältnisses mit einem Referenzwert,
wobei ein vermindertes Verhältnis der Gesamtmenge von *Bifidobacteria* und *Faecalibacterium* zu gramnegativen Proteobakterien (GNPB) gegenüber dem Referenzwert ein Indikator für Zöliakie im Individuum ist.

4. Verfahren nach Anspruch 3 zur Verwendung bei der Überwachung der Reaktion eines Individuums auf die Behandlung von Zöliakie.

## Revendications

1. Produit destiné à être utilisé dans l'amélioration de la santé intestinale d'individus souffrant ou susceptibles de souffrir d'une maladie cœliaque, dans lequel le produit contient des souches de *Lactobacillus rhamnosus SP1* et *Bifidobacterium lactis BLC ;* et éventuellement des prébiotiques et/ou un produit stimulant *Bifidobacteria* et *Faecalibacterium*, ou n'importe quelle combinaison de celles-ci ; et dans lequel le produit augmente le rapport de la quantité totale de *Bifidobacteria* et *Faecalibacterium* aux GNPB (protéobactéries Gram négatives) lorsqu'il est administré à un individu.

2. Produit destiné à être utilisé selon la revendication 1, dans lequel le prébiotique est choisi dans le groupe constitué par les fructo- et galacto-oligosaccharides, les xylo-oligosaccharides, les fibres, et l'avoine.

3. Procédé pour détecter une maladie cœliaque chez un individu, le procédé comprenant :
(a) la détermination du rapport de la quantité totale de *Bifidobacteria* et *Faecalibacterium* aux protéobactéries Gram négatives (GNPB) dans un échantillon fécal d'un individu ; et
(b) la comparaison du rapport déterminé dans l'étape (a) avec une valeur de référence,
dans lequel un rapport de la quantité totale de *Bifidobacteria* et *Faecalibacterium* aux protéobactéries Gram négatives (GNPB) inférieur à la valeur de référence est indicatif d'une maladie cœliaque chez ledit individu.

4. Procédé selon la revendication 3, destiné à être utilisé dans la surveillance de la réponse d'un individu à un traitement d'une maladie cœliaque.
